# EUROPEAN PATENT SPECIFICATION

(11) **EP 1 951 167 B1**
(45) Date of publication and mention of the grant of the patent: **27.01.2010**
(21) Application number: 06837771.2
(22) Date of filing: 16.11.2006
(51) Int. Cl.: A61F 2/84

(54) **Introducer for implantable device**
Einführgerät für eine implantierbare Vorrichtung
Dispositif d'introduction d'un implant

(30) Priority: 16.11.2005 US 737179 P
(43) Date of publication of application: 06.08.2008
(73) Proprietor: William, A. Cook Australia Pty. Ltd., Brisbane, Queensland 4113 (AU); Cook Incorporated, Bloomington, Indiana 47402-0489 (US); William Cook Europe ApS, 4632 Bjaeverskov (DK); THE CLEVELAND CLINIC FOUNDATION, Cleveland, OH 44195 (US)
(72) Inventor: HARTLEY, David, Ernest, Subiaco, Western Australia 6008 (AU); IVANCEV, Krasnodar, S-22221 Lund (SE); GREENBERG, Roy, K., Bratenahl, OH 44108 (US)
(74) Representative: Powell, Stephen David
(86) International application number: PCT/US2006/044494
(87) International publication number: WO 2007/059280

(56) References cited:
- WO-A2-00/76570
- WO-A2-2005/037141
- US-A- 5 617 854

## Description

The present invention relates to an introducer for an implantable device, such as a stent graft, which can be implanted into the vasculature of a human or animal body.

In some procedures for implanting medical devices into a passageway within the human or animal body, it is necessary to insert a guide wire for the device and then to snare or catch the end of the wire by means of an element inserted into the body via a different route.

One such procedure is disclosed in PCT application PCT/US2004/033566 (WO2005/037141) in which a guide wire extends through an indwelling catheter inserted with a first deployment device into the iliac artery. After correct positioning of the guide wire, it is snared by a snare element deployed through the contra-lateral iliac artery. Subsequently a second deployment device is introduced through the contra-lateral iliac artery into the proximal end of the iliac artery and then into the internal iliac artery.

Problems can arise in snaring the end of the guide wire. In particular, when being deployed from the end of the introducer, the guide wire and/or a catheter from which it extends can become entangled around the end of the introducer or the main guide wire of the introducer. Another possible problem is that, when attempting to extend the guide wire into its snaring position, it moves straight ahead into the aorta beyond the junction with the iliac and contralateral iliac arteries; in such cases access by the snare element from the contralateral iliac artery is extremely difficult.

Aspects of the present invention seek to overcome or reduce one or more of the above problems.

According to a first aspect of the present invention, there is provided an introducer for an implantable device comprising a leading end member, a guide catheter, and retaining means, wherein the leading end member has an elongate space for receiving an end portion of the catheter and wherein the retaining means has a first position relative to the leading end member, in which it retains an end portion of the catheter in the elongate space, and a second position relative to the leading member, in which it exposes the end portion of the catheter, and wherein the catheter has a guide wire extending therethrough, the guide wire being capable of being extended from the end portion of the catheter, characterised in that the end portion of the catheter is curved when unconstrained so that it may curve away from said leading end member in the second position of the retaining means.

According to a second aspect of the present invention, there is provided an introduction arrangement for a branched stent graft intended for deployment into the lumen of a vessel having a blind vessel extending therefrom; the branched stent graft having a main tubular body having a distal end and a proximal end with a main lumen therethrough, a side arm extending from the main body and having a side arm lumen therethrough and in fluid communication with the main lumen, the introduction arrangement including an introducer, the introducer having a distal end intended to remain outside a patient in use and a proximal end, the proximal end having a nose cone dilator and an arrangement to retain the branched stent graft distally of the nose cone dilator, the nose cone dilator comprising a longitudinal groove thereon, the branched stent graft being retained on the introducer, and a sheath on the introducer extending over the branched stent graft to the nose cone dilator, an indwelling catheter comprising a pre-curved proximal end, the indwelling catheter extending from the distal end of the introducer through an introducer lumen in the introducer to the branched stent graft, exiting from the introducer lumen at a distal end of the branched stent graft and entering the distal end of the side arm through the side arm lumen to the main lumen and extending out of the proximal end of the branched stent graft to the groove in the nose cone dilator, wherein in a partially retracted position of the sleeve the pre-curved proximal end of the indwelling auxiliary catheter is exposed and in a curved configuration and in an advanced position of the sleeve the curved proximal end of the indwelling auxiliary catheter is in a straightened configuration, extends along the groove in the nose cone and is covered by the sleeve, the indwelling catheter having a graft distally of the nose cone dilator, the nose cone dilator comprising a longitudinal groove thereon, the branched stent graft being retained on the introducer, and a sheath on the introducer extending over the branched stent graft to the nose cone dilator, an indwelling catheter comprising a pre-curved proximal end, the indwelling catheter extending from the distal end of the introducer through an introducer lumen in the introducer to the branched stent graft, exiting from the introducer lumen at a distal end of the branched stent graft and entering the distal end of the side arm through the side arm lumen to the main lumen and extending out of the proximal end of the branched stent graft to the groove in the nose cone dilator, wherein in a partially retracted position of the sleeve the pre-curved proximal end of the indwelling auxiliary catheter is exposed and in a curved configuration and in an advanced position of the sleeve the curved proximal end of the indwelling auxiliary catheter is in a straightened configuration, extends along the groove in the nose cone and is covered by the sleeve, the indwelling catheter having a guide wire extending therethrough, whereby the sheath can be partially withdrawn such that the proximal end of the indwelling auxiliary catheter is in its curved configuration and the guide wire can be extended through the curved end of the indwelling catheter before the sheath is fully withdrawn from the branched stent graft.

The term pre-curved is intended to refer to the proximal end of the auxiliary catheter having a curve set into it so that in an unconstrained or natural state the proximal end is curved but such that in a constrained or held state the proximal end can be straightened.

In accordance with embodiments of the present invention, an introducer for a stent graft such as an iliac branched stent graft, can be introduced with the curved catheter straightened along the groove in the nose cone dilator and then when the introducer is adjacent the aortic bifurcation the sleeve can be withdrawn so that the curved catheter takes up its curved configuration again. An auxiliary guide wire can then be extended to the proximal end of the contralateral iliac artery, where it can be snared without the danger of it being entangled with the nose cone or the main guide wire of the introducer.

The introducer can be supplied and sterilised with the sleeve in the retracted position so that the curved proximal end of the indwelling auxiliary catheter remains in its curved configuration but that during introduction the sleeve can be advanced so that the curved catheter is placed into the straightened configuration so that the introducer can be advanced through the vasculature.

The means to retain the breached stent graft on the introducer may include trigger wires extending to the distal end of the introducer and release arrangements for separate release of the proximal and distal ends of the stent graft from the introducer.

### Brief Description of the Drawing

A preferred embodiment of the present invention will now be described, by way of example only, with reference to the accompanying drawings, of which:
Figure 1 shows an introducer incorporating a catheter according to an embodiment of the present invention;
Figure 2 shows the embodiment of Figure 1 with the end of the catheter exposed;
Figure 3 shows an enlarged cross-sectional view of the proximal end of the introducer as shown in Figure 2;
Figures 4 and 5 show views similar to Figure 3 with the catheter and an associated guide wire in different dispositions;
Figure 6 shows a schematic view of the vasculature of a patient with an introducer deployed into the vasculature; and
Figures 7 and 8 show views similar to Figure 6 of further stages in the deployment and snaring of a catheter of an introducer according to the present invention.

### Detailed Description

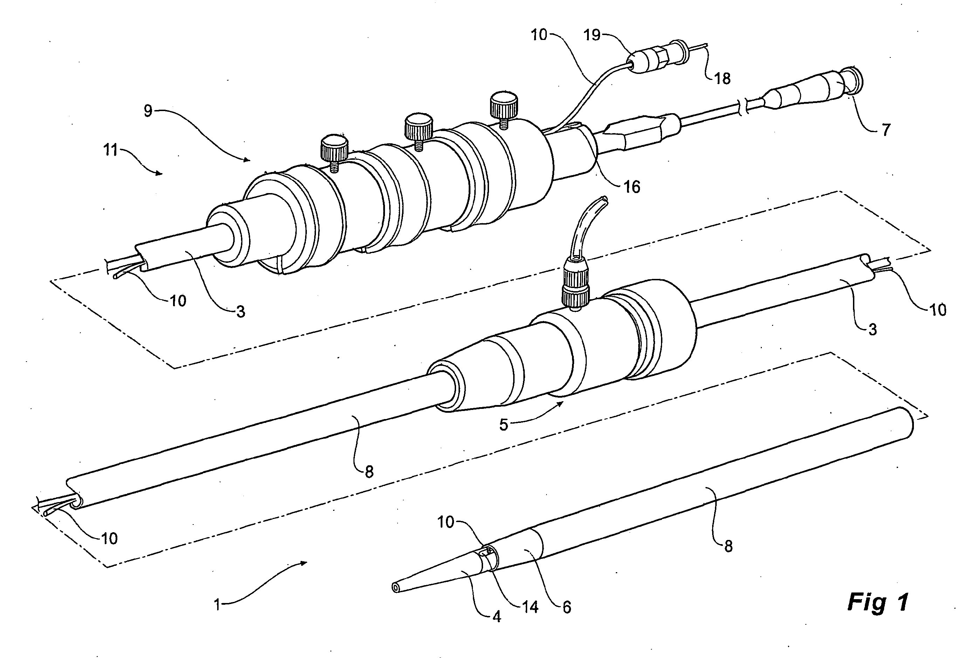

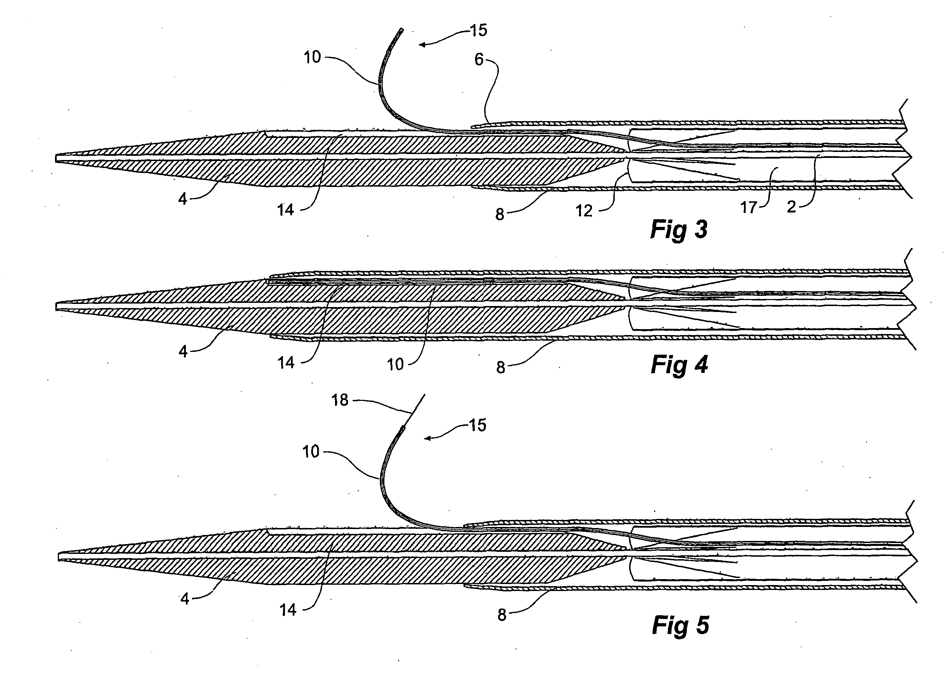

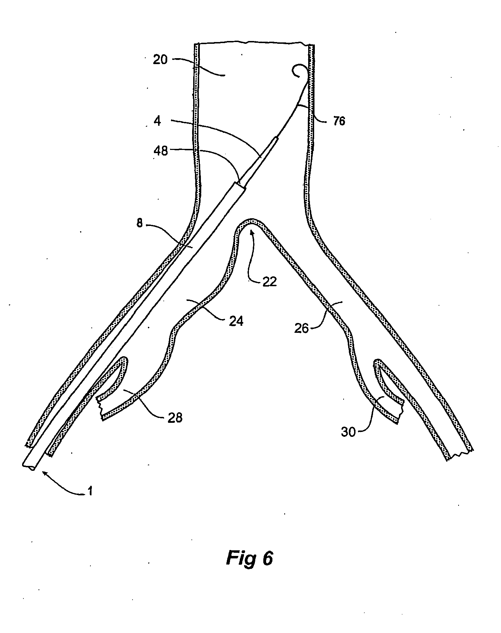

Referring to the drawings, Figure 1 shows an introducer 1 having a delivery catheter 3 which extends from a distal handle 11 to a leading end member in the form of a proximal tapered nose cone dilator 4 longitudinally through the passageway of a sheath or sleeve 8. The introducer sheath 8 extends from a tapered proximal end 6 to a connector valve and sleeve manipulator member 5 attached about the distal end of the sheath. Member 5 includes a silicone disk (not shown) for preventing the backflow of fluids therethrough. The silicone disk includes a slit for the insertion of the nose cone dilator 4 and delivery catheter 3. A stent graft 17, Figure 3 is carried on the delivery catheter 3. Member 5 also includes a side arm to which a polyvinyl tube is connected for introducing and aspirating fluids therethrough. Nose cone dilator 4 includes a tapered proximal end for accessing and dilating a vascular access site over a well-known and commercially available main guide wire 76, Figure 6. Such a guide wire is inserted in a vessel with an introducer needle using, for example, the well-known percutaneous vascular access Seldinger technique. A well-known female Luer lock connector hub 7 is attached at the distal end of the delivery catheter 3 for connection to syringes and other medical apparatus. A set of trigger wire release arrangements generally shown as 9 is on the handle 11 and is used to retain and release the stent graft 17.

An indwelling catheter 10 extends through a lumen of the delivery catheter 3 to the handle and exits the handle in a groove 16 in the handle 11. The indwelling catheter 10 has an auxiliary guide wire 18 extending through it. The nose cone dilator 4 includes a longitudinal groove 14 in which is received the proximal end portion 15 of the indwelling catheter 10.

In the advanced position of the sheath 8 as shown in Figure 1 the proximal end portion 15 of the indwelling catheter 10 is retained in a straight configuration and extends along the length of the groove 14 in the nose cone dilator 4. In this position, the introducer 1 can be deployed through the vasculature of a patient to the junction of the iliac and contralateral iliac arteries with the aorta.

As described so far, the introducer corresponds generally to the introducer disclosed in WO 2005/037141. However, as described below in greater detail with reference to Figures 3 to 5, the end portion 15 of the catheter 10 has an unconstrained shape which is curved. During manufacture, or at some other time before introduction into the vasculature, the end portion is bent, so that it can be said to have a pre-curved configuration.

Thus when the sheath 8 is partially retracted from the dilator 4 over at least part of the groove 14, the end portion 15 is exposed and is no longer constrained so that it adopts its natural curved shape. This causes the end of the catheter 10 to bend away from the dilator 4.

A preferred catheter size is 4.0F (i.e. a diameter of 1.32mm) but sizes between 3.0 and 4.1 F (0.99mm to 1.35mm diameter) also perform well.

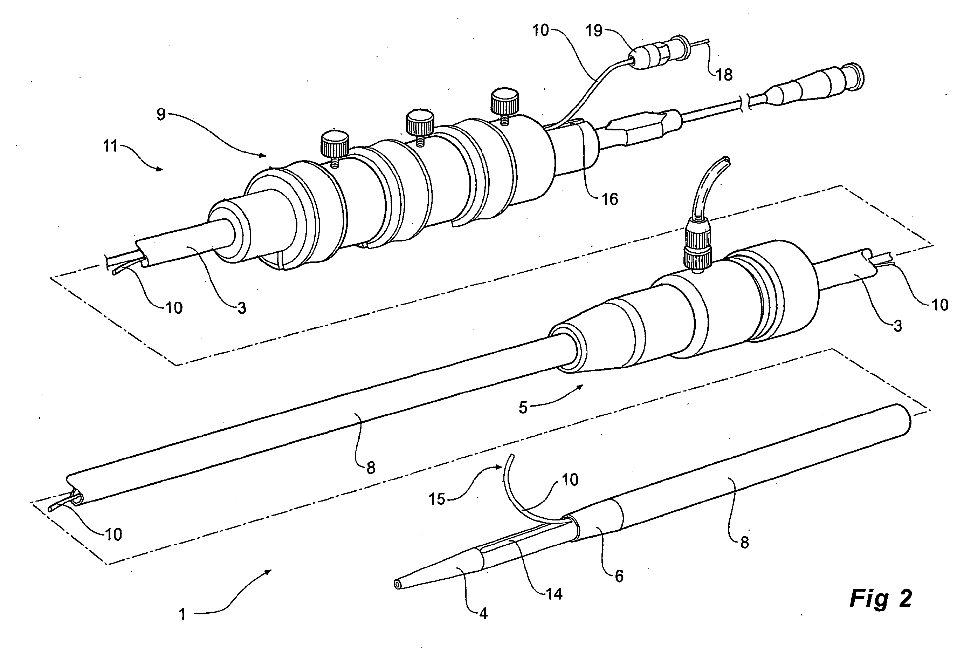

Figure 3 shows an enlarged view of the proximal end of the introducer 1 of Figures 1 and 2 in its configuration during assembly and sterilisation. It also corresponds generally to the configuration of Figure 2. A catheter 2 for the main guide wire 76, Figure 6, extends to the proximal end of the nose cone dilator 4. As in WO 2005/037141, a branched stent graft 17 is retained distally of the nose cone by retention means (not shown) and is covered by the sheath 8. The indwelling catheter 10 extends into the distal or outer end of the branch of the stent graft and out of the proximal end 12 of the stent graft 17 and into the groove 14 in the nose cone 4.

In the retracted position of the sheath 8 as shown in Figure 3, the proximal end portion 15 of the indwelling catheter 10 is in its curved configuration and is free to curve away from the nose cone dilator 4.

Figure 4 shows a view simitar to Figure 3 but showing the introducer in its configuration during deployment, with the sheath 8 advanced. This corresponds to the configuration shown in Figure 1.

As shown in Figure 4, the sheath 8 has been advanced to cover a majority of the nose cone dilator 4 and the pre-curved proximal end 15 of the indwelling catheter 10 has been straightened so that it is in a straight configuration extending along the length of the groove 14 in the nose cone dilator 4. In preferred arrangements, the end of the catheter 10 extends along substantially the entire length of groove 14. In this position, the introducer can be deployed through the vasculature of a patient to the deployment site.

To enable the end portion 15 of catheter 10 to curve away from dilator 4 to a satisfactory extent, it has been found preferable for the length of groove 14 to be longer than 25mm which was the length of the groove in the dilator of WO 2005/037141. Thus groove 14 typically has a length of from 30 to 60mm, more preferably from 40 to 50mm and most preferably 45mm. A preferred width is 2mm. The length of the curved portion 15 at the end of catheter 10 is typically 20 to 40mm, more preferably 25 to 35mm and most preferably 30mm. In its retracted position as shown in Figure 3, the sheath typically exposes half to all of the length of groove 14, more preferably three fifths to three quarters of the length, and most preferably two thirds.

The catheter is preferably made of a material with some degree of resilience, such as polyethylene, nylon or polyurethane, and preferably with a reinforcing braid. Although curvature of the end portion 15 through an angle of approximately 120° as shown in Fig. 3 is acceptable, it is preferable to curve it through a range of between 180° and 225°. To achieve this, a loop is produced in a former of relatively stiff wire with a curvature through 270° to 360° (i.e. up to one complete turn). The catheter 10 is then threaded over the loop in the wire and the combination is heated thoroughly in steam. The combination is then quenched in water and the catheter is then removed from the former in which condition the end portion 15 has a curvature through typically 270° around a diameter of 12mm or less.

Guide wire 18 is then threaded through catheter 10, which causes the curved portion 15 to open up slightly, but preferably still retaining a curvature of approximately 210° around a diameter of, typically, 15mm.

In use, after the introducer 1 is in a desired position, as will be discussed in relation to figures 6 to 8, the sheath 8 is withdrawn as shown in Figure 5 so that the indwelling catheter 10 can resume the curved shape of its proximal end 15 so that it is directed away from the nose cone dilator 4. Its auxiliary guide wire 18 can then be advanced to extend from the catheter 10 so that it can be snared as discussed below.

Figures 6 to 8 show schematic views of the vasculature of a patient with an introducer 1 deployed into the vasculature.

Figure 6 corresponds generally to Figure 1 of WO 2005/037141 and shows aorta 20 extending down to an aortic bifurcation 22 from which extend iliac arteries 24 and 26. An internal iliac artery 28 extends from the iliac artery 24 and an internal iliac artery 30 extends from the contralateral iliac artery 26.

As described in WO 2005/037141, if an aneurysm in the iliac artery 24 extends down and includes the opening to the internal iliac artery 28, it will be necessary for the deployment of a stent graft 17 into the common iliac artery, to have a fenestration or side arm on the stent graft so that an extension piece can be placed into the internal iliac artery 28. As shown in Figure 6, the introducer 1 has been introduced over its main guide wire 76 and positioned so that the nose cone 4 is extending up into the aorta 20 and so that the proximal end 48 of the sheath 8 extends past the aortic bifurcation 22.

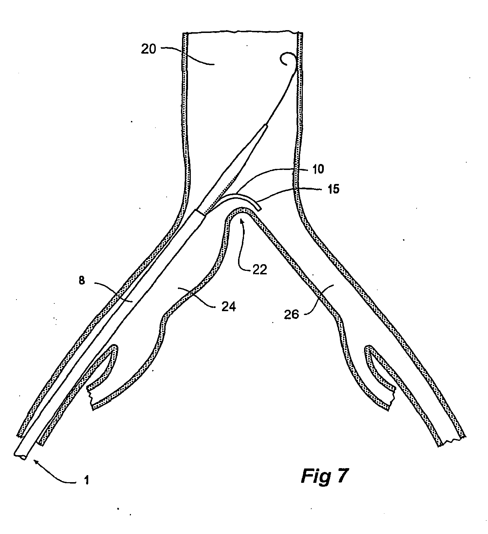

In the next stage as shown in Figure 7, the sheath 8 is partially withdrawn so that the proximal end portion 15 of the indwelling catheter 10 takes up its original curved shape as discussed above in detail in relation to Figure 5 and, in particular, the curved proximal end of the indwelling catheter is directed towards the contralateral iliac artery 26.

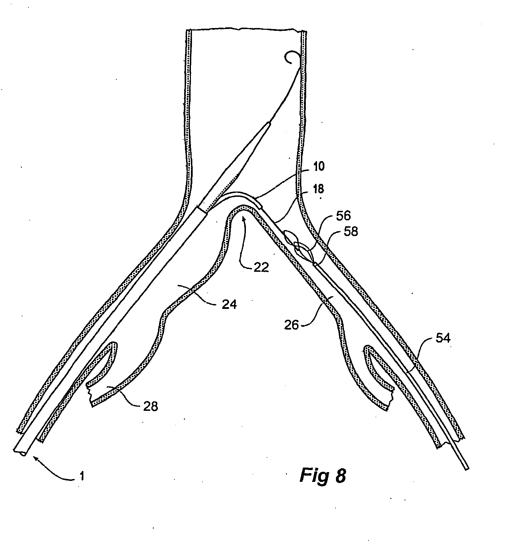

As shown in Figure 8, which corresponds generally to Figure 2A of WO 2005/037141, guide wire 18 can then be extended from the catheter 10 to extend down the contralateral iliac artery and a snare catheter 54 with a snare arrangement 56 at its proximal end 58 can be used to snare the guide wire 18. By visually monitoring and appropriately manipulating the position and orientation of the end portion 15 of catheter 10, it is possible to ensure that guide wire 18 also avoids unnecessary entanglements and is deployed in the desired direction. The manipulations of position and amount of curvature of the end portion of the catheter can be produced by repeated retractions and advances of sheath 8 until catheter 10 is correctly deployed.

Monitoring the end portion 15 of catheter 10 also enables the location of groove 14 to be determined. The introducer 1, or an appropriate part thereof, can be rotated so that, when required, the groove 14 is on the side of the nose cone dilator 4 facing the artery 26.

In subsequent stages of the deployment of the stent grant 17 into the iliac artery, the sheath 8 can be withdrawn to deploy the stent graft into the iliac artery and a further introducer can be deployed over the guide wire 18 from the contralateral artery to pass through the side arm or fenestration in the stent graft and enable direction of a stent graft extension into the internal iliac artery 28. WO 2005/037141 discloses examples of such methods by which extensions can be placed into such arteries as discussed above.

An advantage of the above described arrangement is that during deployment neither the proximal end portion 15 of the catheter 10 nor its auxiliary guide wire 18 becomes wrapped around nose cone dilator 4 or any other parts of the introducer 1. In addition, it can be ensured that the catheter 10 is in the correct location and pointing in the right direction before the guide wire 18 is extended along the contralateral iliac artery 26. By deploying the guide wire in the relatively confined space of the artery 26, there is a better chance of ensnaring it in a problem-free manner than in the aorta 20, where it was frequently deployed in prior art arrangements.

Various modifications may be made to the above-described embodiment. For example the length of the end portion 15 which is curved may be varied as desired. It is possible for the last few millimetres of the catheter 10 to be straight, so that the actual curved portion extends close to but not completely up to the end. The degree of curvature imparted to end portion 15 may vary along the length of the curve. A typical minimum radius of curvature is 4mm.

In another modification, catheter 10 does not extend fully into groove 14 and it is the end of the auxiliary guide wire 18 itself which has a curved shape when unconstrained. Thus retraction of sheath 8 immediately releases guide wire 18 into bifurcation 22. This retains the advantage of avoiding entanglement, but there is a greater possibility that the wire will deploy in an unintended direction. In further modifications, catheter 10 may not extend into groove 14 at all or, indeed, may be completely omitted with only the guide wire 18 extending from handle 11.

Although described in connection with the implantation of a branched stent graft as described in WO 2005/037141, introducers according to the present invention may be used to deploy any type of implantable device at any location where a guide element needs to be precisely positioned and/or orientated, whether for a subsequent snaring procedure or otherwise.

## Claims

1. An introducer (1) for an implantable device (17) comprising a leading end member (4), a guide catheter (10), and retaining means (8), wherein the leading end member has an elongate space (14) for receiving an end portion (15) of the catheter and wherein the retaining means has a first position relative to the leading end member, in which it retains an end portion of the catheter in the elongate space, and a second position relative to the leading member, in which it exposes the end portion of the catheter, and wherein the catheter has a guide wire (18) extending therethrough, the guide wire being capable of being extended from the end portion of the catheter, **characterised in that** the end portion (15) of the catheter (10) is curved when unconstrained so that it may curve away from said leading end member in the second position of the retaining means.

2. An introducer according to claim 1, wherein the elongate space is a longitudinal groove (14) in the surface of the leading end member (4).

3. An introducer according to claim 2, wherein the elongate space (14) has a length of between 30 and 60mm.

4. An introducer according to any preceding claim, wherein the curved end portion of the catheter (10) has a length of between 20 and 40mm.

5. An introducer according to any preceding claim, wherein the implantable device (17) is a branched stent graft and the catheter (10) extends through the branch of the graft.

6. An introduction arrangement for a branched stent graft (17) intended for deployment into the lumen (24) of a vessel having a blind vessel (28) extending therefrom; the branched stent graft (17) having a main tubular body having a distal end and a proximal end with a main lumen therethrough, a side arm extending from the main body and having a side arm lumen therethrough and in fluid communication with the main lumen, the introduction arrangement including an introducer (1), the introducer having a distal end intended to remain outside a patient in use and a proximal end, the proximal end having a nose cone dilator (4) and an arrangement to retain the branched stent graft distally of the nose cone dilator, the nose cone dilator comprising a longitudinal groove (14) thereon, the branched stent graft being retained on the introducer, and a sheath (8) on the introducer extending over the branched stent graft to the nose cone dilator, an indwelling catheter (10) comprising a pre-curved proximal end (15), the indwelling catheter extending from the distal end of the introducer through an introducer lumen in the introducer to the branched stent graft, exiting from the introducer lumen at a distal end of the branched stent graft and entering the distal end of the side arm through the side arm lumen to the main lumen and extending out of the proximal end of the branched stent graft to the groove in the nose cone dilator, wherein in a partially retracted position of the sleeve the pre-curved proximal end of the indwelling auxiliary catheter is exposed and in a curved configuration and in an advanced position of the sleeve the curved proximal end of the indwelling auxiliary catheter is in a straightened configuration, extends along the groove in the nose cone and is covered by the sleeve, the indwelling catheter having a guide wire extending therethrough, whereby the sheath can be partially withdrawn such that the proximal end of the indwelling auxiliary catheter is in its curved configuration and the guide wire can be extended through the curved end of the indwelling catheter before the sheath is fully withdrawn from the branched stent graft.

## Patentansprüche

1. Einführgerät (1) für eine implantierbare Vorrichtung (17), das ein führendes Endelement (4), einen Führungskatheter (10) und ein Haltemittel (8) umfasst, wobei das führende Endelement einen länglichen. Raum (14) zur Aufnahme eines Endabschnitts (15) des Katheters aufweist und wobei das Haltemittel eine erste Position relativ zum führenden Endelement, in der es einen Endabschnitt des Katheters in dem länglichen Raum hält, und eine zweite Position relativ zu dem führenden Element aufweist, in der es den Endabschnitt des Katheters freilegt, und worin der Katheter einen Führungsdraht (18) aufweist, der sich durch ihn hindurch erstreckt, wobei der Führungsdraht vom Endabschnitt des Katheters aus ausgefahren werden kann, **dadurch gekennzeichnet, dass** der Endabschnitt (15) des Katheters (10) im nicht eingezwängten Zustand gebogen ist, so dass er sich vom führenden Endelement in der zweiten Position des Haltemittels weg biegen kann.

2. Einführgerät nach Anspruch 1, worin der längliche Raum eine Längsrille (14) in der Oberfläche des führenden Endabschnitts (4) ist.

3. Einführgerät nach Anspruch 2, worin der längliche Raum (14) eine Länge zwischen 30 und 60 mm aufweist.

4. Einführgerät nach einem der vorhergehenden Ansprüche, worin der gekrümmte Endabschnitt des Katheters (10) eine Länge zwischen 20 und 40 mm aufweist.

5. Einführgerät nach einem der vorhergehenden Ansprüche, worin die implantierbare Vorrichtung (17) eine verzweigte Stent-Graftprothese ist und der Katheter (10) sich durch den Ast der Graftprothese erstreckt.

6. Einführungsanordnung für eine verzweigte Stent-Graftprothese (17) zur Ablage im Lumen (24) eines Gefäßes mit einem sich davon erstreckenden Blindgefäß (28); wobei die verzweigte Stent-Graftprothese (17) einen röhrenförmigen Hauptkörper mit einem distalen Ende und einem proximalen Ende mit einem durch sie hindurch gehenden Hauptlumen aufweist, wobei sich ein Seitenarm vom Hauptkörper erstreckt und ein durch ihn hindurch gehendes Seitenarmlumen in Flüssigkeitsverbindung mit dem Hauptlumen aufweist, wobei die Einführungsanordnung ein Einführgerät (1) aufweist, wobei das Einführgerät ein distales Ende, das außerhalb des Patienten bleiben soll, und ein proximales Ende aufweist, wobei das proximale Ende einen Nasenkonusdilatator (4) und eine Anordnung aufweist, die die verzweigte Stent-Graftprothese distal vom Nasenkonusdilatator hält, wobei der Nasenkonusdilatator eine Längsrille (14) darauf, wobei die verzweigte Stent-Graftprothese auf dem Einführgerät gehalten wird, und eine Schleuse (8) auf dem Einführgerät umfasst, die sich über die verzweigte Stent-Graftprothese bis zum Nasenkonusdilatator erstreckt, wobei ein Verweilkatheter (10) ein vorgebogenes proximales Ende (15) umfasst, wobei sich der Verweilkatheter vom distalen Ende des Einführgeräts durch ein Einführungslumen im Einführgerät bis zur verzweigten Stent-Graftprothese erstreckt, aus dem Einführungslumen an einem distalen Ende der verzweigten Stent-Graftprothese austritt und in das distale Ende des Seitenarms durch das Seitenarmlumen zum Hauptlumen eintritt und sich aus dem proximalen Ende der verzweigten Stent-Graftprothese zur Rille im Nasenkonusdilatator erstreckt, worin das vorgebogene proximale Ende des Hilfsverweilkatheters in einer teilweise zurückgezogenen Position der Schleuse freigelegt ist und in einer gebogenen Konfiguration vorliegt und das gebogene proximale Ende des Hilfsverweilkatheters in einer vorgeschobenen Position der Schleuse in einer gestreckten Konfiguration vorliegt, sich entlang der Rille im Nasenkonus erstreckt und von der Schleuse abgedeckt wird, wobei der Verweilkatheter einen sich durch ihn hindurch erstreckenden Führungsdraht aufweist, wodurch die Schleuse teilweise zurückgezogen werden kann, so dass das proximale Ende des Hilfsverweilkatheters in seiner gebogenen Konfiguration vorliegt und der Führungsdraht durch das gebogene Ende des Verweilkatheters ausgefahren werden kann, bevor die Schleuse vollständig aus der verzweigten Stent-Graftprothese herausgezogen wird.

## Revendications

1. Dispositif d'introduction (1) pour un implant (17), comprenant un organe d'extrémité avant (4), un cathéter de guidage (10) et un moyen de retenue (8), l'organe d'extrémité avant ayant un espace allongé (14) pour recevoir une portion d'extrémité (15) du cathéter, le moyen de retenue ayant une première position par rapport à l'organe d'extrémité avant, dans laquelle il retient une portion d'extrémité du cathéter dans l'espace allongé, et une deuxième position par rapport à l'organe avant, dans laquelle il expose la portion d'extrémité du cathéter, le cathéter ayant un fil-guide (18) s'étendant à travers lui, le fil-guide étant en mesure d'être étendu depuis la portion d'extrémité du cathéter, **caractérisé en ce que** la portion d'extrémité (15) du cathéter (10) est courbée lorsqu'elle n'est pas contrainte de sorte qu'elle puisse s'écarter en se courbant depuis ledit organe d'extrémité avant dans la deuxième position du moyen de retenue.

2. Dispositif d'introduction selon la revendication 1, dans lequel l'espace allongé est une gorge longitudinale (14) dans la surface de l'organe d'extrémité avant (4).

3. Dispositif d'introduction selon la revendication 2, dans lequel l'espace allongé (14) a une longueur comprise entre 30 et 60 mm.

4. Dispositif d'introduction selon l'une quelconque des revendications précédentes, dans lequel la portion d'extrémité courbe du cathéter (10) a une longueur comprise entre 20 et 40 mm.

5. Dispositif d'introduction selon l'une quelconque des revendications précédentes, dans lequel l'implant (17) est une endoprothèse vasculaire à greffer à branche et le cathéter (10) s'étend à travers la branche de l'endoprothèse.

6. Agencement d'introduction pour une endoprothèse vasculaire à greffer à branche (17) destinée à être déployée dans la lumière (24) d'un vaisseau ayant un vaisseau borgne (28) s'étendant depuis celle-ci ; l'endoprothèse vasculaire à greffer à branche (17) ayant un corps principal tubulaire ayant une extrémité distale et une extrémité proximale avec une lumière principale à travers lui, un bras latéral s'étendant depuis le corps principal et ayant une lumière de bras latéral le traversant et en communication fluidique avec la lumière principale, l'agencement d'introduction comportant un dispositif d'introduction (1), le dispositif d'introduction ayant une extrémité distale destinée à rester à l'extérieur d'un patient pendant l'utilisation et une extrémité proximale, l'extrémité proximale ayant un dispositif de dilatation conique à nez (4) et un agencement pour retenir l'endoprothèse vasculaire à greffer à branche en position distale par rapport au dispositif de dilatation conique à nez, le dispositif de dilatation conique à nez comprenant une gorge longitudinale (14) sur celui-ci, l'endoprothèse vasculaire à greffer à branche étant retenue sur le dispositif d'introduction et une gaine (8) sur le dispositif d'introduction s'étendant par-dessus l'endoprothèse vasculaire à greffer à branche jusqu'au dispositif de dilatation conique à nez, un cathéter à demeure (10) comprenant une extrémité proximale précourbée (15), le cathéter à demeure s'étendant depuis l'extrémité distale du dispositif d'introduction à travers une lumière de dispositif d'introduction dans le dispositif d'introduction jusqu'à l'endoprothèse vasculaire à greffer à branche, sortant de la lumière du dispositif d'introduction à une extrémité distale de l'endoprothèse à greffer vasculaire à branche et entrant dans l'extrémité distale du bras latéral à travers la lumière du bras latéral jusqu'à la lumière principale et s'étendant hors de l'extrémité proximale de l'endoprothèse vasculaire à greffer à branche jusqu'à la gorge dans le dispositif de dilatation conique à nez, l'extrémité proximale précourbée du cathéter auxiliaire à demeure étant exposée et dans une configuration courbée dans une position partiellement rentrée du manchon, et dans une position avancée du manchon, l'extrémité proximale courbée du cathéter auxiliaire à demeure étant dans une configuration droite, s'étendant le long de la gorge dans le cône à nez et étant recouverte par le manchon, le cathéter à demeure ayant un fil-guide s'étendant à travers lui, la gaine pouvant être partiellement retirée de telle sorte que l'extrémité proximale du cathéter auxiliaire à demeure soit dans sa configuration courbée et que le fil-guide puisse être étendu à travers l'extrémité courbée du cathéter à demeure avant que la gaine ne soit complètement retirée de l'endoprothèse vasculaire à greffer à branche.
